# EUROPEAN PATENT SPECIFICATION

(11) **EP 3 489 216 B1**
(45) Date of publication and mention of the grant of the patent: **31.08.2022**
(21) Application number: 17830897.9
(22) Date of filing: 11.07.2017
(51) Int. Cl.: C07C 68/06, C07C 69/96

(54) **PROCESS FOR PRODUCING CHLOROFORMATE COMPOUND**
VERFAHREN ZUR HERSTELLUNG EINER CHLOROFORMIATVERBINDUNG
PROCÉDÉ DE FABRICATION DE COMPOSÉ CHLOROFORMIATE

(30) Priority: 21.07.2016 JP 2016143647
(43) Date of publication of application: 29.05.2019
(73) Proprietor: Kaneka Corporation, Osaka-shi, Osaka 530-8288 (JP)
(72) Inventor: YASUKOUCHI, Hiroaki, Takasago-shi Hyogo 676-8688 (JP); FUNABASHI, Makoto, Takasago-shi Hyogo 676-8688 (JP); NISHIYAMA, Akira, Takasago-shi Hyogo 676-8688 (JP); TAKEDA, Toshihiro, Takasago-shi Hyogo 676-8688 (JP); MITSUDA, Masaru, Takasago-shi Hyogo 676-8688 (JP)
(74) Representative: Hoffmann Eitle
(86) International application number: PCT/JP2017/025220
(87) International publication number: WO 2018/016377

(56) References cited:
- CN-A- 101 245 001
- CN-A- 103 408 427
- JP-A- H0 296 551
- JP-A- 2011 006 367
- JP-A- 2011 026 308
- JP-A- 2012 067 030
- JP-A- 2015 528 434
- US-A1- 2005 065 361
- US-B1- 6 392 079

## Description

### TECHNICAL FIELD

The present invention relates to a process for producing a chloroformate compound using a flow reactor.

### BACKGROUND ART

A chloroformate compound is very useful as a raw material and an intermediate for pharmaceuticals, agrochemicals because of its high reactivity. The development of a production process capable of synthesizing such a chloroformate compound safely in a high yield and at low cost has been desired. As a method for producing the chloroformate compound, a method of allowing an alcohol compound to react with phosgene gas or phosgene generated in a system from diphosgene or triphosgene in a batch reactor is generally adopted. Specifically, a method is known in which phosgene gas is dissolved in tetrahydrofuran in a batch reactor, and then 9-fluorenylmethanol is added thereto to produce a corresponding 9-fluorenylmethyl chloroformate (Patent Document 1). Furthermore, a method is also known in which phosgene gas is dissolved in dichloromethane in a batch reactor, and then tributylphosphine and 9-fluorenylmethanol are added thereto to produce a corresponding 9-fluorenylmethyl chloroformate (Patent Document 2). In addition, a method is known in which a solution of triphosgene in dichloromethane or chloroform is prepared in a batch reactor, an amine such as diisopropylethylamine, 4-dimethylaminopyridine, or pyridine is added thereto to generate phosgene in a system, and the generated phosgene is allowed to react with 9-fluorenylmethanol to produce 9-fluorenylmethyl chloroformate (Patent Documents 3 and 4). It is also known to produce an alkyl chloroformate by first mixing phosgene or triphosgene with an alcohol, expelling surplus phosgene using a nitrogen gas, and then reacting the obtained mixture solution and an amine (Patent Document 5). As in the method of Patent Document 5, a method of expelling surplus phosgene gas after mixing phosgene and an alcohol is beneficial in that the theoretical use amount of phosgene is equimolar to the amount of the alcohol, and hence the theoretical amount of an amine hydrochloride generated can also be reduced to an equimolar amount to the alcohol. However, this method is dangerous because the phosgene gas is released outside the system. Patent Document 7 describes a continuous process for the preparation of certain monofunctional aromatic chloroformates (MAC) by mixing and reacting (i) an aqueous caustic solution; (ii) a carbonyl chloride; (iii) at least one monofunctional hydroxyaromatic compound; and (iv) at least one inert organic solvent. Patent Document 8 describes a method for the preparation of alky/aryl chloroformates directly from alcohols and triphosgene.

### PRIOR ART DOCUMENT

### PATENT DOCUMENT

Patent Document 1: JPH 2-96551 (A)
Patent Document 2: JPH 2-129151 (A)
Patent Document 3: CN 103408427 (A)
Patent Document 4: CN 101245001 (A)
Patent Document 5: JP 2012-67030 (A)
Patent Document 6: JP 2011-6367 (A)
Patent Document 7: US 6,392,079 (B1)
Patent Document 8: US 2005/065361 (A1)

### SUMMARY OF THE INVENTION

### PROBLEMS TO BE SOLVED BY THE INVENTION

However, when these methods are carried out industrially, it is necessary to hold a very large amount of highly toxic phosgene gas in the reactor, which is very dangerous. In addition, when phosgene is generated in the system, the operation of controlling the generation rate is complicated and there is a risk of getting out of control. It is also dangerous to expel the surplus phosgene gas. Therefore, in order to carry out the manufacture safely, special facilities sufficiently taking countermeasures against phosgene leakage are required, and hence costs for the facilities increase.

A flow (continuous production) method is known as a reaction method different from a batch method. As an example of producing a chloroformate compound by the flow (continuous production) method, a method is known in which a mixed solution of an aqueous solution containing sodium hydroxide and potassium hydroxide and 1,1-bis-(4-hydroxyphenyl)cyclohexane is mixed and reacted continuously in a microchannel with a solution obtained by dissolving phosgene gas in dichloromethane to produce a corresponding bischloroformate compound (Patent Document 6). However, even when using this method, since the work of dissolving the phosgene gas in the organic solvent in advance is necessary, the dangerousness remains the same.

Accordingly, it is an object of the present invention to provide a method for mass-producing a chloroformate compound, which is useful as a raw material and intermediate for pharmaceuticals, agrochemicals and the like, safely and at a high yield.

### SOLUTIONS TO THE PROBLEMS

As a result of intensive studies for achieving the above object, the inventors have found that unlike the case of dissolving phosgene gas, when triphosgene, which is a compound having low toxicity, is dissolved in advance, preliminary work can be carried out safely without fear of phosgene leakage. In addition, the inventors have found that when the triphosgene thus dissolved and a solution that is separately prepared and contains an amine and an alcohol compound are mixed and reacted in a flow reactor, a phosgene which is generated by contact between the triphosgene and the amine is rapidly consumed by the alcohol compound, whereby the reaction can be carried out while stably preventing the increase in concentration of highly toxic phosgene in the reaction solution (that is, while keeping the risk of phosgene leakage low). The invention has been achieved based on such findings. A production process of the present invention is advantageous in that a special phosgene gas generator is not required, and exhaust gas treating equipment such as a scrubber is not also required; in that since the flow reactor system is generally space-saving, safety measures can be easily taken, for example, by covering the entire system, and even if phosgene leaks, it is possible to minimize damage and thus significantly improve safety for workers; and in that expelling of surplus phosgene is not required.

Specifically, the present invention is as follows.
[1] A process for producing a chloroformate compound, comprising mixing and reacting a solution of triphosgene with a solution comprising an amine and an alcohol compound in a flow reactor.
[2] The process according to [1], wherein the amine is tributylamine.
[3] The process according to [1] or [2], wherein the amine is used in an amount of 0.8 to 3 equivalents relative to an amount of the alcohol compound.
[4] The process according to any of [1] to [3], wherein the solution comprises an aromatic hydrocarbon solvent or an ether solvent.
[5] The process according to any of [1] to [4], wherein the solution comprises toluene or tetrahydrofuran.
[6] The process according to any of [1] to [5], wherein the triphosgene is used in an amount of 0.3 to 1 equivalent relative to an amount of the alcohol compound.
[7] The process according to any of [1] to [6], wherein a flow channel of the flow reactor has a cross-sectional area of 10 mm² to 30 cm².
[8] The process according to any of [1] to [7], wherein a reaction temperature in the flow channel of the flow reactor is 60°C or lower.
[9] The process according to any of [1] to [8], wherein a retention time in the flow channel of the flow reactor is within 10 minutes.

### EFFECT OF THE INVENTION

According to the present invention, it is possible to produce a large amount of chloroformate compound, which is useful as a raw material and intermediate for pharmaceuticals, agrochemicals and the like, safely and at a high yield.

### MODE FOR CARRYING OUT THE INVENTION

In the present invention, a solution of triphosgene which is a compound having low toxicity is mixed and reacted with a solution containing an amine (hereinafter also referred to as an amine compound) and an alcohol compound in a flow reactor to produce a chloroformate compound. When triphosgene is dissolved in a first solvent to prepare a solution of triphosgene, dissolution work can be conducted safely unlike the case of dissolving phosgene gas. Furthermore, since the triphosgene is mixed and reacted with the solution containing an amine compound and an alcohol compound in a flow reactor, when phosgene is generated by contact between the triphosgene and the amine compound, this phosgene is rapidly consumed by the alcohol compound in a micro-space, whereby a desired product can be produced while preventing accumulation of phosgene and maintaining the safety.

One molecule of the triphosgene corresponds to 3 molecules of phosgene. Therefore, the amount of use of the triphosgene is, for example, 0.3 mol or more, preferably 0.35 mol or more, and is, for example, 10 mol or less, preferably 5 mol or less, more preferably 2 mol or less, further preferably 1 mol or less, particularly preferably 0.8 mol or less, relative to 1 mol of the alcoholic hydroxyl group of the alcohol compound. When expressed in terms of equivalents, the amount of use of the triphosgene is, for example, 0.3 equivalent or more, preferably 0.35 equivalent or more, and is, for example, 10 equivalents or less, preferably 5 equivalents or less, more preferably 2 equivalents or less, further preferably 1 equivalent or less, particularly preferably 0.8 equivalent or less, relative to the amount of the alcohol compound.

The first solvent is not particularly limited as long as triphosgene dissolves in the solvent and the solvent is not involved in the reaction. Examples of the first solvent include an aliphatic hydrocarbon solvent such as n-hexane, cyclohexane, or methylcyclohexane; an aromatic hydrocarbon solvent such as benzene, toluene, or xylene; an ether solvent such as diethyl ether, diisopropyl ether, tetrahydrofuran (THF), methyltetrahydrofuran, methyl tert-butyl ether (MTBE), 4-methyltetrahydropyran, 1,4-dioxane, or cyclopentyl methyl ether (CPME); a halogen-containing solvent such as dichloromethane, chloroform, 1,1,1,-trichloroethane, or chlorobenzene; an ester solvent such as ethyl acetate, propyl acetate, or butyl acetate; a ketone solvent such as acetone, methyl ethyl ketone, or methyl isobutyl ketone; a nitrile solvent such as acetonitrile, propionitrile, or butyronitrile; and an amide solvent such as N,N-dimethylformamide, N,N-dimethylacetamide, or N-methylpyrrolidone. These solvents may be used singly or in combination of two or more, and there are no particular restrictions on the mixing ratio of the solvents. The aromatic hydrocarbon solvent such as benzene, toluene, or xylene; and the ether solvent such as diethyl ether, diisopropyl ether, tetrahydrofuran (THF), methyltetrahydrofuran, methyl tert-butyl ether (MTBE), or cyclopentyl methyl ether (CPME) are preferable, and toluene or tetrahydrofuran (THF) is more preferable.

The amount of the first solvent may be suitably determined as long as triphosgene can be dissolved and a product does not precipitate during the reaction with the amine compound and the alcohol compound. The amount of the first solvent is, for example, 0.8 part by mass or more, preferably 3 parts by mass or more, more preferably 5 parts by mass or more, and is, for example, 200 parts by mass or less, preferably 100 parts by mass or less, more preferably 70 parts by mass or less, relative to 1 part by mass of triphosgene.

The amine compound is preferably a tertiary amine, and specific examples the amine compound include trimethylamine, triethylamine, tripropylamine, tributylamine, tripentylamine, trihexylamine, trioctylamine, diethylamine, diisopropylethylamine, dimethylethylamine, dicyclohexylmethylamine, N-methylpyrrolidine, N-methylmorpholine, 1,8-diazabicyclo[5,4,0]undec-7-ene, pyridine, 2-picoline, 3-picoline, 2,6-lutidine, collidine, 4-dimethylaminopyridine, quinoline, imidazole, N-methylimidazole and the like. These amines may be used singly or in combination of two or more. When using in combination of two or more amines, there are no particular restrictions on the mixing ratio. The tertiary amine such as tripropylamine, tributylamine, trihexylamine, trioctylamine, diisopropylethylamine, 1,8-diazabicyclo[5,4,0]undec-7-ene, N-methylimidazole, or the like is preferable, and a trialkylamine is more preferable. In view of excellent reactivity and difficulty of precipitating an amine hydrochloride, the tertiary amine (particularly, noncyclic trialkylamine) is preferable.

In the method for producing a chloroformate by generating phosgene from triphosgene and an amine compound in the system and reacting the generated phosgene with an alcohol compound, formation of an amine hydrochloride as a by-product concurrently with the progress of the reaction cannot be prevented. This amine hydrochloride generally has low solubility in an organic solvent and precipitates out of the solution, and thus the reaction solution often forms slurry. The biggest disadvantage of the flow reactor is that when the reaction solution forms slurry, line is clogged, so that the solution cannot flow in the line. To apply this reaction to the flow reactor, it is desirable that a solid does not precipitate out of the reaction solution. When the amine and the solvent are appropriately combined, the clogging of the line can be preferably prevented. More preferably, several combinations of the amine and the solvent can suppress the precipitation of the amine hydrochloride out of the reaction solution and also contribute to improvement of the yield of a chloroformate reaction.

Focusing on the difficulty of precipitating amine hydrochlorides, it is particularly preferable that the amine compound is a trialkylamine having 9 to 40 carbon atoms. The number of carbon atoms of the trialkylamine is preferably 10 or more, more preferably 12 or more, and is preferably 30 or less, more preferably 24 or less. As the trialkylamines, tripropylamine, tributylamine, trihexylamine, and trioctylamine are preferable, and tributylamine is most preferable.

The using amount of the amine compound is, for example, 0.1 mol or more, preferably 0.5 mol or more, more preferably 0.8 mol or more, further preferably 1.3 mol or more, and is, for example, 10 mol or less, preferably 5 mol or less, more preferably 3 mol or less, further preferably 2 mol or less, relative to 1 mol of the alcoholic hydroxyl group of the alcohol compound. When expressed in terms of equivalents, the using amount of the amine compound is, for example, 0.1 equivalents or more, preferably 0.5 equivalents or more, more preferably 0.8 equivalents or more, further preferably 1.3 equivalents or more, and is, for example, 10 equivalents or less, preferably 5 equivalents or less, more preferably 3 equivalents or less, further preferably 2 equivalents or less, relative to the amount of the alcohol compound.

The using amount of the amine compound is 0.1 mol or more, preferably 1 mol or more, more preferably 1.5 mol or more, further preferably 3 mol or more, and is, for example, 10 mol or less, preferably 6 mol or less, more preferably 4 mol or less, relative to 1 mol of triphosgene. A too large amount of the amine compound is not preferable because the amount of the amine hydrochloride generated may become large, resulting in increased possibility of precipitation, and is not also preferable in terms of post-treatment. A too small amount of the amine compound is not preferable in terms of the progress of the reaction because the generation of phosgene is delayed.

The alcohol compound is not particularly limited as long as it has a structure in which a hydroxyl group is bonded to a carbon atom. For example, the alcohol compound may be a compound in which a hydroxyl group is bonded to a non-aromatic hydrocarbon group or a compound having a phenolic hydroxyl group. In addition, the alcohol compound may be a compound having one hydroxyl group in the molecule thereof such as a primary alcohol, a secondary alcohol, a tertiary alcohol, or a phenol; a compound having two hydroxyl groups in the molecule thereof such as a diol or a catechol; a compound having three hydroxyl groups in the molecule thereof such as a triol or a benzenetriol; and a compound having four or more hydroxyl groups in the molecule thereof such as a sugar or a nucleic acid. Furthermore, the alcohol compound may be an optical active compound.

Specific examples of the alcohol compound include methanol, ethanol, n-propanol, isopropanol, n-butanol, isobutanol, sec-butanol, tert-butanol, n-pentanol, 2-pentanol, n-octanol, n-dodecanol, n-octadecanol, 9-fluorenylmethanol, L-menthol, cyclopropanol, cyclobutanol, cyclopentanol, cyclohexanol, cyclopropylmethanol, 2,2,2-trifluoroethanol, 4,4,4-trifluorobutanol, 2-methoxyethanol, 2-phenethyl alcohol, allyl alcohol, 5-hexenol, adamantan-1-ol, adamantan-2-ol, benzyl alcohol, 1-phenethyl alcohol, methyl hydroxyacetate, methyl L-lactate, dimethyl L-malate, N-(tert-butoxycarbonyl)-L-alaninol, N-(benzyloxycarbonyl)-L-phenylalaninol, N-(tert-butoxycarbonyl)-L-serine methyl ester, (S)-ethyl mandelate, 2-hydroxypropionitrile, N-benzyl-3-pyrrolidinol, N-(tert-butoxycarbonyl)-3-piperidinol, 5-methyl-1,3-dioxolane-2-one-4-methanol, L-alaninol, glycidol, (R)-3-quinuclidinol, methyl (S)-3-hydroxybutanoate, methyl (S) -2-methyl-3-hydroxypropionate, trans-N-(tert-butoxycarbonyl)-4-hydroxy-L-proline methyl ester, ethylene glycol, 1,2-butanediol, 1,3-butanediol, 1,4-butanediol, 1,2-cyclopentanediol, 1,2-cyclohexanediol, 1,4-cyclohexanediol, L-tartaric acid dimethyl, 1,1-bis-(4-hydroxydiphenyl)cyclohexane, 1,3,5-cyclohexanetriol, phenol, 1-naphthol, 1,2-catechol, glycerin, 1,3,5-benzenetriol, polyethylene glycol, glucose, fructose, trehalose, xylitol, ascorbic acid, cellulose, thymine, uracil, and the like. 9-fluorenyl methanol, L-menthol, and phenol are preferable, and 9-fluorenyl methanol and L-menthol are more preferable.

Desirably, the amine compound and the alcohol compound are, for example, dissolved in a second solvent to form a solution, and the solution is mixed with a solution of triphosgene in a flow reactor. As the second solvent, the same as those exemplified above for the first solvent can be used. Preferable examples of the second solvent are also the same as those of the first solvent. The first solvent and the second solvent may be the same or different.

The amount of the second solvent can be determined within the range in which both the amine compound and the alcohol compound can be dissolved. Specifically, the amount of the second solvent is, for example, 10 parts by weight or more, preferably 50 parts by weight or more, more preferably 100 parts by weight or more, and is, for example, 1000 parts by weight or less, preferably 500 parts by weight or less, more preferably 200 parts by weight or less, relative to 100 parts by weight of the alcohol compound.

The total amount of the first solvent and the second solvent can be suitably determined within the range in which an amine hydrochloride and chloroformate compound can be dissolved. In particular, since the amine hydrochloride tends to precipitate easily, it is practical to specify a relationship between the amount of the amine compound serving as an indicator of the amount of this amine hydrochloride generated and the total amount of the first and second solvents. The weight ratio of the amine compound and the total amount of the first and second solvents (the former/the latter) is, for example, 1/100 or more, preferably 2/100 or more, more preferably 2.5/100 or more, and is, for example, 100/100 or less, preferably 60/100 or less, more preferably 40/100 or less.

In the present invention, a solvent (third solvent) other than the solvents exemplified for the first and second solvents may coexist as necessary. The concentration of the third solvent in the whole solvent is, for example, 10 wt% or less, preferably 5 wt% or less, more preferably 1 wt% or less.

In the present invention, the above-mentioned reaction is carried out in a flow reactor. The flow reactor is an apparatus having two or more raw material feeding ports, a mixing unit to mix the fed raw materials, and a reactor unit (sometimes referred to as a tubular reaction unit, a retention line or the like) in which a mixed solution flows. The reactor unit can have different shape such as a micro flow tube having a coil structure, a plate structure in which a micro flow channel is engraved on a plate, or a laminated structure in which these plates are stacked. The reaction progresses while the mixed solution flows in the reactor unit. A microreactor, a cyclone-shaped reactor, and a laminated microfluidic chip are all included in the flow reactor of the present invention. The raw materials are fed in the form of a liquid (including a solution), and the liquid is usually transferred using a pump such as a diaphragm pump, a syringe pump, a plunger pump or the like. The raw material feeding ports, the mixing unit, and the reactor unit are liquid-tightly connected.

The solution containing triphosgene, the amine compound (may be an amine compound-containing solution), and the alcohol compound (may be an alcohol compound-containing solution) are all fed as liquids from the raw material feeding ports to the mixing unit. The solution containing triphosgene, the amine compound, and the alcohol compound may be fed from different feeding ports. Alternatively, a mixed solution containing the amine compound, the alcohol compound and a solvent is prepared in advance, and this mixed solution and the solution containing triphosgene may be fed from different feeding ports. According to these feed procedures, an increase in concentration of phosgene can be suppressed, and safety is therefore ensured, as compared with the case where a mixed solution containing triphosgene and the amine compound is prepared in advance, or where a mixed solution containing triphosgene and the alcohol compound is prepared in advance.

A known mixer can be used for the mixing unit. For example, a T-shape mixer (including a T-shape tube) and a Y-shape mixer (including a Y-shape tube) can be used as a mixer having two inflow channels and one outflow line. A mixing unit having three or more inflow channels can also be used as the mixing unit (mixer). The mixing unit (mixer) may be a static-type mixer, or a helix-type mixer.

The number of the mixing units (mixers) is suitably determined depending on the number of inflow channels and the number of raw material feeding ports of one mixing unit. For example, in the case where three raw material feeding ports are present, and the solution containing triphosgene, the amine compound (may be an amine compound-containing solution) and the alcohol compound (may be an alcohol compound-containing solution) are separately fed, two mixing units may be provided to mix the amine compound and the alcohol compound in a first mixing unit and mix the amine-alcohol mixture solution and the solution containing triphosgene in a next mixing unit (first method). In the case where two raw material feeding ports are present and one mixer with two inflow channels is used, the amine-alcohol mixture solution may be preliminarily prepared, and this preliminarily prepared solution and the solution containing triphosgene may be introduced separately from the raw material feeding ports to mix them in the mixing unit (second method). According to the first method and the second method, since the alcohol compound coexists when phosgene is generated by contact between the triphosgene and the amine compound, the phosgene immediately reacts with the alcohol compound, so that accumulation of phosgene can be prevented, and hence the reaction can proceed safely in a micro-space.

The mixed solution prepared in the mixing unit is fed to the reactor unit, and the reaction proceeds while the mixed solution flows in the reactor unit. The cross-sectional area of the flow channel of the mixing unit and the reactor unit is, for example, 10 µm² or more, preferably 1 mm² or more, more preferably 10 mm² or more, and is, for example, 300 cm² or less, preferably 70 cm² or less, more preferably 30 cm² or less.

In this reaction, the reaction time is controlled by the length of the reactor unit (retention line) and the flow rate. The length of the reactor unit is, for example, 1 cm or more, preferably 10 cm or more, more preferably 1 m or more, and is, for example, 500 m or less, preferably 300 m or less, more preferably 100 m or less. The flow rate is, for example, 0.01 mL/min or more, preferably 0.1 mL/min or more, more preferably 0.5 mL/min or more, and is, for example, 30 L/min or less, preferably 20 L/min or less, more preferably 10 L/min or less. The linear velocity is, for example, 0.005 m/min or more, preferably 0.05 m/min or more, more preferably 0.5 m/min or more, and is, for example, 180 m/min or less, preferably 120 m/min or less, more preferably 60 m/min or less. The reaction time (retention time) is, for example, within 30 minutes, preferably within 20 minutes, more preferably within 15 minutes, most preferably within 10 minutes, and is, for example, 5 minutes or longer, preferably 3 minutes or longer, more preferably 1 minute or longer.

The reaction temperature can be set within the range of from the freezing point to the boiling point of the solvent. The reaction temperature is, for example, 100°C or lower, preferably 60°C or lower, more preferably 40°C or lower, and is, for example, -50°C or higher, preferably -30°C or higher, more preferably -10°C or higher, and may be 20°C or higher. When a chloroformate reaction is carried out by a batch method, the reaction temperature at the time of generation of phosgene generally needs to be equal to or lower than the boiling point (8°C) of phosgene from the viewpoint of safety. However, when using the flow reactor, the reaction can be carried out in a closed micro-space and hence can be carried out safely even at a temperature of about 20 to 60°C (especially 20 to 40°C), whereby energy savings can be attained. The temperature of the mixing unit and the temperature on the upstream side of the mixing unit may also be appropriately set, and, for example, may be the same as the reaction temperature. These temperatures may also be lower than the reaction temperature in order to improve efficiency of heat removal.

The materials of the mixing unit and the reactor unit are not particularly limited and may be appropriately selected depending on needs for solvent resistance, pressure resistance, heat resistance, or the like. For example, a metal such as stainless steel, Hastelloy, titanium, copper, nickel and aluminum; a glass; a ceramics; and a resin such as PEEK resin, silicone resin, and fluororesin can be used.

The reaction solution flowing out from the reactor unit is appropriately worked-up as necessary. For example, after quenching the reaction solution with an aqueous solution containing an acid such as hydrofluoric acid, hydrochloric acid, hydrobromic acid, hydroiodic acid, sulfuric acid, phosphoric acid or the like, an organic solvent such as ethyl acetate or toluene may be optionally added to extract a desired product. The amount of the acid aqueous solution to be used for quenching is not particularly limited, and usually, is 0.1 parts by weight or more, preferably 0.5 parts by weight or more, more preferably 1 part by weight or more, and is 100 parts by weight or less, preferably 50 parts by weight or less, more preferably 20 parts by weight or less, relative to 1 part by weight of the alcohol compound. An organic solvent such as ethyl acetate or toluene may be optionally added, and quenching may be carried out in a two-layer system of water-organic solvent. In addition, the extract can also be washed with acidic water, inorganic salt water or water as necessary. The reaction solvent and the extraction solvent are distilled away from the resultant extract by an operation such as heating under reduced pressure or the like, whereby a desired product is obtained.

The desired product thus obtained has sufficient purity to be used in a subsequent step. The purity may be further increased by using a commonly used purification technique such as crystallization, fractional distillation, column chromatography, or the like.

### EXAMPLES

Hereinbelow, the present invention will be more specifically described in examples, but the present invention is not limited to these descriptions.

### Reference Example 1

Each of 1 g of various amines shown in the following Table 1 was added to 10 mL of each solvent shown in the following Table 1 to prepare a 10% concentration (weight/volume) solution. An n-propanol solution of hydrochloric acid (hydrochloric acid concentration: 34% by weight) was added to this solution until reaching 1.1 equivalents (= molar amount of hydrogen chloride/molar amount of amine), and the mixture was stirred for 1 hour at room temperature. After stirring, whether solids were precipitated or not was visually confirmed. In the table, "Good" means that no solid was precipitated, and "Poor" means that solid was precipitated.

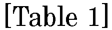

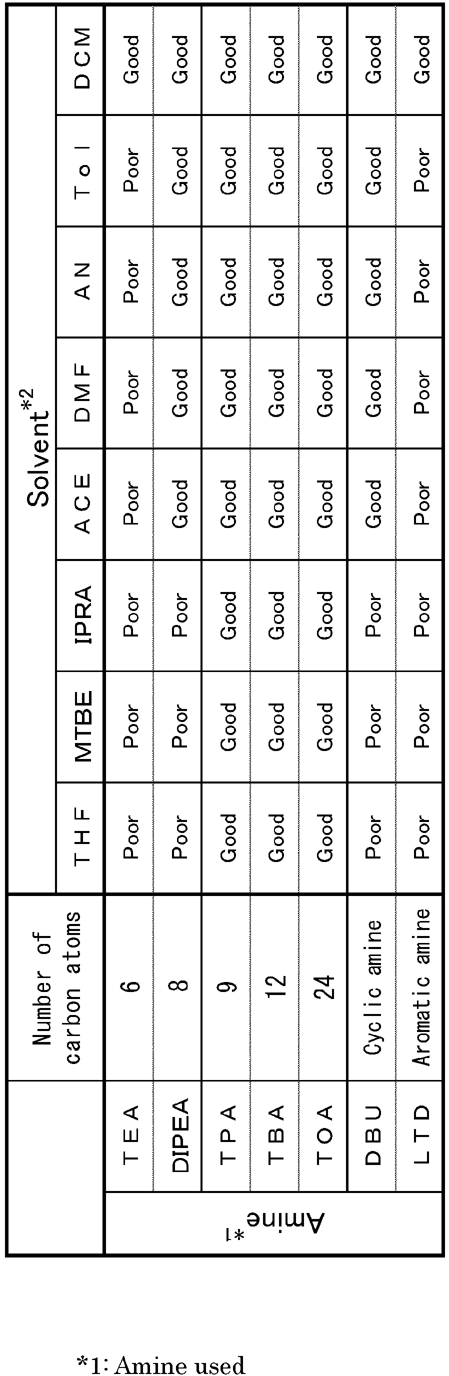

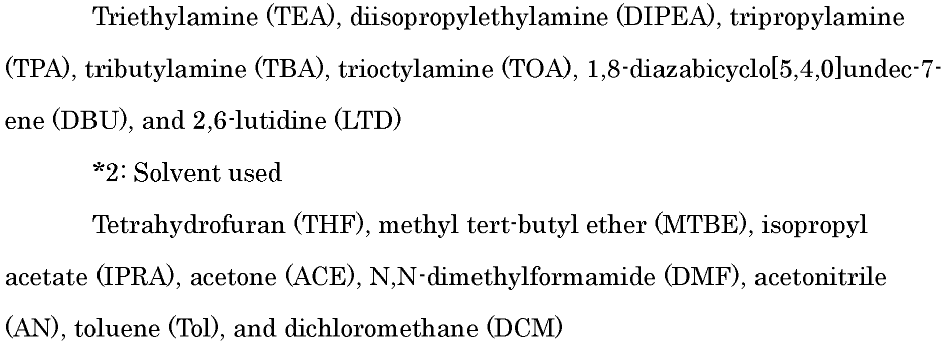

### (1) Production of 9-fluorenylmethyl chloroformate

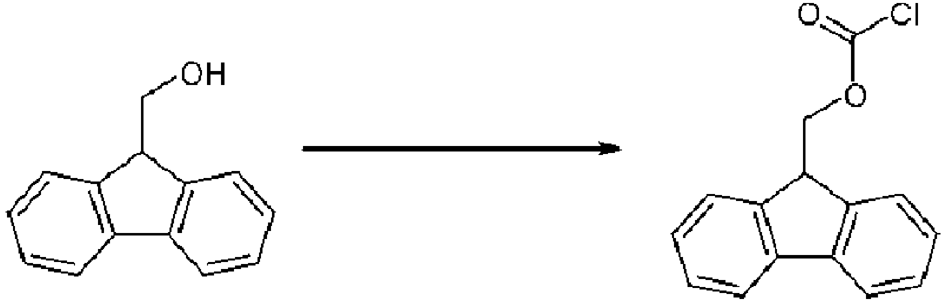

In the following Examples 1 to 5, Comparative Examples 1 and 2, and Reference Example 2, 9-fluorenylmethyl chloroformate was produced from 9-fluorenylmethanol and triphosgene, and the product was quantified by the HPLC method to calculate the yield. HPLC conditions were as follows.

Column: Daicel CHIRALPAC IA (250 × 4.6 mm)
Mobile phase: hexane/ethanol = 85/15 (volume ratio)
Flow rate: 1.0 ml/min
Detection wavelength: UV 254 nm
Column temperature: 30°C
Retention time: 9-fluorenylmethyl chloroformate; 4.4 minutes, 9-fluorenylmethanol; 5.8 minutes

### Example 1

To 0.61 g of triphosgene, 25.49 g of toluene was added to prepare a homogeneous solution, and the resulting solution was referred to as solution A. To 1.42 g of tributylamine and 1.00 g of 9-fluorenylmethanol, 22.81 g of toluene was added to prepare a homogeneous solution, and the resulting solution was referred to as solution B. AT-shape mixer (inner diameter: 2 mm, material: polytetrafluoroethylene (PTFE)) and a retention line (inner diameter of tube: 2 mm, material: polytetrafluoroethylene (PTFE)) were placed in a constant-temperature bath at 0°C. Then, the solution A and the solution B were each transferred using a diaphragm pump (manufactured by KNF Japan Co. Ltd.) at a rate of 2 ml/min (linear velocity: 127 cm/min) and mixed by the T-shape mixer, and the resulting mixture was allowed to flow in the retention line for 1 minute to perform a reaction. The reaction solution was quenched while stirring with 34.85 g of a 13% phosphoric acid aqueous solution in a flask. After separation, 66.24 g of an organic layer containing 1.19 g of 9-fluorenylmethyl chloroformate was obtained (yield: 90%). No crystals precipitated during the reaction, and the reaction solution was a clear solution.

### Example 2

To 0.61 g of triphosgene, 25.49 g of toluene was added to prepare a homogeneous solution, and the resulting solution was referred to as solution A. To 1.42 g of tributylamine and 1.00 g of 9-fluorenylmethanol, 22.81 g of toluene was added to prepare a homogeneous solution, and the resulting solution was referred to as solution B. A T-shape mixer (inner diameter: 2 mm, material: polytetrafluoroethylene (PTFE)) and a retention line (inner diameter of tube: 2 mm, material: polytetrafluoroethylene (PTFE)) were placed in a constant-temperature bath at 0°C. Then, the solution A and the solution B were each transferred using a diaphragm pump (manufactured by KNF Japan Co. Ltd.) at a rate of 2 ml/min (linear velocity: 127 cm/min) and mixed by the T-shape mixer, and the resulting mixture was allowed to flow in the retention line for 4 minutes to perform a reaction. The reaction solution was quenched while stirring with 34.85 g of a 13% phosphoric acid aqueous solution in a flask. After separation, 71.85 g of an organic layer containing 1.29 g of 9-fluorenylmethyl chloroformate was obtained (yield: 98%). No crystals precipitated during the reaction, and the reaction solution was a clear solution.

### Example 3

To 0.91 g of triphosgene, 14.00 g of toluene was added to prepare a homogeneous solution, and the resulting solution was referred to as solution A. To 2.12 g of tributylamine and 1.50 g of 9-fluorenylmethanol, 11.50 g of THF was added to prepare a homogeneous solution, and the resulting solution was referred to as solution B. A T-shape mixer (inner diameter: 2 mm, material: polytetrafluoroethylene (PTFE)) and a retention line (inner diameter of tube: 2 mm, material: polytetrafluoroethylene (PTFE)) were placed in a constant-temperature bath at 30°C. Then, the solution A and the solution B were each transferred using a diaphragm pump (manufactured by KNF Japan Co. Ltd.) at a rate of 2 ml/min (linear velocity: 127 cm/min) and mixed by the T-shape mixer, and the resulting mixture was allowed to flow in the retention line for 2 minutes to perform a reaction. The reaction solution was quenched while stirring with 52.28 g of a 13% phosphoric acid aqueous solution in a flask. After separation, 35.48 g of an organic layer containing 1.77 g of 9-fluorenylmethyl chloroformate was obtained (yield: 90%). No crystals precipitated during the reaction, and the reaction solution was a clear solution.

### Example 4

To 1.36 g of triphosgene, 14.00 g of toluene was added to prepare a homogeneous solution, and the resulting solution was referred to as solution A. To 2.12 g of tributylamine and 1.50 g of 9-fluorenylmethanol, 11.50 g of THF was added to prepare a homogeneous solution, and the resulting solution was referred to as solution B. AT-shape mixer (inner diameter: 2 mm, material: polytetrafluoroethylene (PTFE)) and a retention line (inner diameter of tube: 2 mm, material: polytetrafluoroethylene (PTFE)) were placed in a constant-temperature bath at 30°C. Then, the solution A and the solution B were each transferred using a diaphragm pump (manufactured by KNF Japan Co. Ltd.) at a rate of 2 ml/min (linear velocity: 127 cm/min) and mixed by the T-shape mixer, and the resulting mixture was allowed to flow in the retention line for 2 minutes to perform a reaction. The reaction solution was quenched while stirring with 52.28 g of a 13% phosphoric acid aqueous solution in a flask. After separation, 36.21 g of an organic layer containing 1.89 g of 9-fluorenylmethyl chloroformate was obtained (yield: 96%). No crystals precipitated during the reaction, and the reaction solution was a clear solution.

### Example 5

To 0.61 g of triphosgene, 24.81 g of toluene was added to prepare a homogeneous solution, and the resulting solution was referred to as solution A. To 1.42 g of tributylamine and 1.00 g of 9-fluorenylmethanol, 22.81 g of toluene was added to prepare a homogeneous solution, and the resulting solution was referred to as solution B. AT-shape mixer (inner diameter: 2 mm, material: polytetrafluoroethylene (PTFE)) and a retention line (inner diameter of tube: 2 mm, material: polytetrafluoroethylene (PTFE)) were placed in a constant-temperature bath at 40°C. Then, the solution A and the solution B were each transferred using a diaphragm pump (manufactured by KNF Japan Co. Ltd.) at a rate of 2 ml/min (linear velocity: 127 cm/min) and mixed, and the resulting mixture was allowed to flow in the retention line for 2 minutes to perform a reaction. The reaction solution was quenched while stirring with 34.85 g of a 13% phosphoric acid aqueous solution in a flask. After separation, 59.40 g of an organic layer containing 1.24 g of 9-fluorenylmethyl chloroformate was obtained (yield: 94%). No crystals precipitated during the reaction, and the reaction solution was a clear solution.

### Comparative Example 1

In a four-necked flask, 4.26 g of tributylamine and 35.03 g of toluene were placed and then cooled to -5°C. Next, 1.83 g of triphosgene was dissolved in 5.50 g of toluene to prepare a triphosgene solution, the triphosgene solution was added into the four-necked flask at a rate of keeping the internal temperature at -5°C while stirring, and the mixture was stirred for 3 hours. Thereafter, 3.00 g of 9-fluorenylmethanol was dissolved in 105 g of toluene to prepare a solution, the solution was added into the four-necked flask at a rate of keeping the internal temperature at -5°C while stirring, and the mixture was stirred for 1 hour. After stirring, this reaction solution was transferred into 104.55 g of a 13% phosphoric acid aqueous solution, separation was performed after quenching, and 154.33 g of an organic layer containing 2.36 g of 9-fluorenylmethyl chloroformate was obtained (yield: 60%).

### Comparative Example 2

In a four-necked flask, 3.00 g of 9-fluorenylmethanol was dissolved in 4.26 g of tributylamine and 140.01 g of toluene, and then cooled to -5°C. Next, 1.83 g of triphosgene was dissolved in 5.50 g of toluene to prepare a triphosgene solution, the triphosgene solution was added into the four-necked flask at a rate of keeping the internal temperature at -5°C while stirring, and the mixture was stirred for 3 hours. After stirring, this reaction solution was transferred into 104.55 g of a 13% phosphoric acid aqueous solution, separation was performed after quenching, and 156.04 g of an organic layer containing 3.06 g of 9-fluorenylmethyl chloroformate was obtained (yield: 77%).

### Reference Example 2

In a flask, 0.91 g of triphosgene was placed, and dissolved by adding 15 g of toluene to prepare a solution. To this solution, 1.50 g of 9-fluorenylmethanol was added, and the mixture was stirred at 20°C for 2 days. As a result of quantifying the reaction solution after stirring for 2 days, the reaction solution containing 0.49 g of 9-fluorenylmethyl chloroformate was obtained (yield: 25%). Based on the fact that the reaction product was generated, it is understood that triphosgene was decomposed, whereby phosgene was generated in the system.

### (2) Production of L-menthyl chloroformate

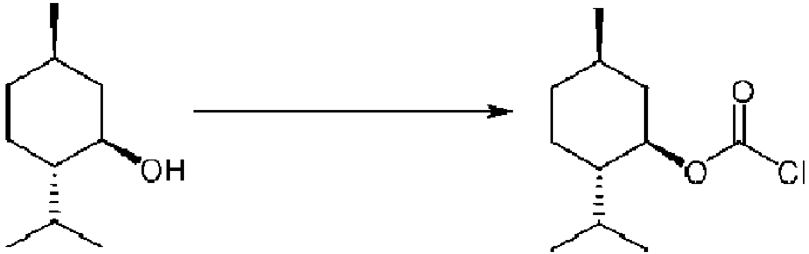

In the following Example 6, menthyl chloroformate was produced from L-menthol and triphosgene, and the product was quantified by the HPLC method to calculate the yield. HPLC conditions were as follows.

Column: Nacalai COSMOSIL 5C18-AR-II (250 × 4.6 mm)
Mobile phase A: 0.1% phosphoric acid aqueous solution
Mobile phase B: acetonitrile
Flow rate: 1.0 ml/min
Detection wavelength: UV 210 nm
Column temperature: 40°C
Retention time: menthyl chloroformate: 28.1 minutes

### Gradient conditions

| Time (min) | Mobile phase A (%) | Mobile phase B (%) |
|---|---|---|
| 0 | 90 | 10 |
| 10 | 40 | 60 |
| 22 | 20 | 80 |
| 27 | 20 | 80 |
| 30 | 10 | 90 |
| 30.1 | 10 | 90 |

### Example 6

To 2.28 g of triphosgene, 20.26 g of toluene was added to prepare a homogeneous solution, and the resulting solution was referred to as solution A. To 5.34 g of tributylamine and 3.00 g of L-menthol, 12.00 g of toluene was added to prepare a homogeneous solution, and the resulting solution was referred to as solution B. AT-shape mixer (inner diameter: 2 mm, material: polytetrafluoroethylene (PTFE)) and a retention line (inner diameter of tube: 2 mm, material: polytetrafluoroethylene (PTFE)) were placed in a constant-temperature bath at 30°C. Then, the solution A and the solution B were each transferred using a diaphragm pump (manufactured by KNF Japan Co. Ltd.) at a rate of 2 ml/min (linear velocity: 127 cm/min) and mixed by the T-shape mixer, and the resulting mixture was allowed to flow in the retention line for 1 minute to perform a reaction. The reaction solution was quenched while stirring with 75.00 g of a 13% phosphoric acid aqueous solution in a flask. After separation, 58.32 g of an organic layer containing 4.15 g of menthyl chloroformate was obtained (yield: 99%). No crystals precipitated during the reaction, and the reaction solution was a clear solution.

### (3) Production of phenyl chloroformate

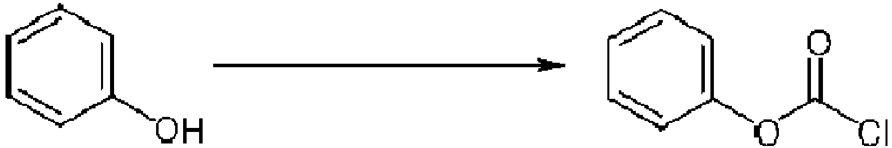

In the following Example 7, phenyl chloroformate was produced from phenol and triphosgene, and the product was quantified by the HPLC method to calculate the yield. HPLC conditions were as follows.

Column: Daicel CHIRALPAC IA (250 × 4.6 mm)
Mobile phase: hexane/isopropanol = 97/3 (volume ratio)
Flow rate: 1.0 ml/min
Detection wavelength: UV 254 nm
Column temperature: 30°C
Retention time: phenyl chloroformate; 4.0 minutes, phenol; 9.5 minutes

### Example 7

To 3.78 g of triphosgene, 21.70 g of toluene was added to prepare a homogeneous solution, and the resulting solution was referred to as solution A. To 8.86 g of tributylamine and 3.00 g of phenol, 12.00 g of toluene was added to prepare a homogeneous solution, and the resulting solution was referred to as solution B. AT-shape mixer (inner diameter: 2 mm, material: polytetrafluoroethylene (PTFE)) and a retention line (inner diameter of tube: 2 mm, material: polytetrafluoroethylene (PTFE)) were placed in a constant-temperature bath at 20°C. Then, the solution A and the solution B were each transferred using a diaphragm pump (manufactured by KNF Japan Co. Ltd.) at a rate of 2 ml/min (linear velocity: 127 cm/min) and mixed by the T-shape mixer, and the resulting mixture was allowed to flow in the retention line for 1 minute to perform a reaction. The reaction solution was quenched while stirring with 83.63 g of a 13% phosphoric acid aqueous solution in a flask. After separation, 60.36 g of an organic layer containing 2.89 g of phenyl chloroformate was obtained (yield: 58%). No crystals precipitated during the reaction, and the reaction solution was a clear solution.

## Claims

1. A process for producing a chloroformate compound, comprising mixing and reacting a solution of triphosgene with a solution comprising an amine and an alcohol compound in a flow reactor.

2. The process according to claim 1, wherein the amine is tributylamine.

3. The process according to claim 1 or 2, wherein the amine is used in an amount of 0.8 to 3 equivalents relative to an amount of the alcohol compound.

4. The process according to any of claims 1 to 3, wherein the solution comprises an aromatic hydrocarbon solvent or an ether solvent.

5. The process according to any of claims 1 to 4, wherein the solution comprises toluene or tetrahydrofuran.

6. The process according to any of claims 1 to 5, wherein the triphosgene is used in an amount of 0.3 to 1 equivalent relative to an amount of the alcohol compound.

7. The process according to any of claims 1 to 6, wherein a flow channel of the flow reactor has a cross-sectional area of 10 mm² to 30 cm².

8. The process according to any of claims 1 to 7, wherein a reaction temperature in the flow channel of the flow reactor is 60°C or lower.

9. The process according to any of claims 1 to 8, wherein a retention time in the flow channel of the flow reactor is within 10 minutes.

## Patentansprüche

1. Verfahren zur Herstellung einer Chloroformiatverbindung, umfassend das Mischen und Umsetzen einer Lösung von Triphosgen mit einer Lösung, die ein Amin und eine Alkoholverbindung umfasst, in einem Durchflussreaktor.

2. Verfahren gemäß Anspruch 1, bei dem das Amin Tributylamin ist.

3. Verfahren gemäß Anspruch 1 oder 2, bei dem das Amin in einer Menge von 0,8 bis 3 Äquivalenten, bezogen auf die Menge der Alkoholverbindung, verwendet wird.

4. Verfahren gemäß mindestens einem der Ansprüche 1 bis 3, bei dem die Lösung ein aromatisches Kohlenwasserstofflösungsmittel oder ein Etherlösungsmittel umfasst.

5. Verfahren gemäß mindestens einem der Ansprüche 1 bis 4, bei dem die Lösung Toluol oder Tetrahydrofuran umfasst.

6. Verfahren gemäß mindestens einem der Ansprüche 1 bis 5, bei dem das Triphosgen in einer Menge von 0,3 bis 1 Äquivalent, bezogen auf die Menge der Alkoholverbindung, verwendet wird.

7. Verfahren gemäß mindestens einem der Ansprüche 1 bis 6, bei dem ein Durchflusskanal des Durchflussreaktors eine Querschnittsfläche von 10 mm² bis 30 cm² aufweist.

8. Verfahren gemäß mindestens einer der Ansprüche 1 bis 7, bei dem eine Reaktionstemperatur in dem Durchflusskanal des Durchflussreaktors 60 °C oder weniger beträgt.

9. Verfahren gemäß mindestens einem der Ansprüche 1 bis 8, bei dem eine Verweilzeit in dem Durchflusskanal des Durchflussreaktors innerhalb von 10 Minuten liegt.

## Revendications

1. Procédé de production d'un composé de chloroformiate, comprenant le mélange et la réaction d'une solution de triphosgène avec une solution comprenant une amine et un composé alcool dans un réacteur à écoulement.

2. Procédé selon la revendication 1, dans lequel l'amine est la tributylamine.

3. Procédé selon la revendication 1 ou 2, dans lequel l'amine est utilisée en une quantité allant de 0,8 à 3 équivalents par rapport à une quantité du composé alcool.

4. Procédé selon l'une quelconque des revendications 1 à 3, dans lequel la solution comprend un solvant hydrocarboné aromatique ou un solvant éther.

5. Procédé selon l'une quelconque des revendications 1 à 4, dans lequel la solution comprend du toluène ou du tétrahydrofurane.

6. Procédé selon l'une quelconque des revendications 1 à 5, dans lequel le triphosgène est utilisé en une quantité allant de 0,3 à 1 équivalent par rapport à une quantité du composé alcool.

7. Procédé selon l'une quelconque des revendications 1 à 6, dans lequel un canal d'écoulement du réacteur d'écoulement présente une surface de section transversale allant de 10 mm² à 30 cm².

8. Procédé selon l'une quelconque des revendications 1 à 7, dans lequel une température de réaction dans le canal d'écoulement du réacteur d'écoulement est inférieure ou égale à 60 °C.

9. Procédé selon l'une quelconque des revendications 1 à 8, dans lequel un temps de rétention dans le canal d'écoulement du réacteur d'écoulement est inférieur à 10 minutes.
